(19) Europäisches Patentamt
European Patent Office
Office européen des brevets

(11) **EP 3 910 359 A1**

(12) **EUROPEAN PATENT APPLICATION**

(43) Date of publication:
**17.11.2021 Bulletin 2021/46**

(51) Int Cl.:
*G01R 33/565* (2006.01)   *G01R 33/56* (2006.01)
*A61B 5/055* (2006.01)   *G06N 3/08* (2006.01)
*G01R 33/561* (2006.01)

(21) Application number: **20174072.7**

(22) Date of filing: **12.05.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **SOLIMAN, Abraam**
  **5656 AE Eindhoven (NL)**
• **VAN GEMERT, Jeroen**
  **5656 AE Eindhoven (NL)**
• **DE WEERDT, Elwin**
  **5656 AE Eindhoven (NL)**

(74) Representative: **Philips Intellectual Property & Standards**
**High Tech Campus 5**
**5656 AE Eindhoven (NL)**

(54) **MACHINE LEARNING BASED DETECTION OF MOTION CORRUPTED MAGNETIC RESONANCE IMAGING DATA**

(57)    The present disclosure relates to a method for detecting motion corrupted magnetic resonance imaging data comprising: receiving (201) acquired k-space data of an object, reconstructing (203) an image from the acquired k-space data, generating (205) reconstructed k-space data from the reconstructed image, determining (207) delta k-space data as a difference between the acquired k-space data and the reconstructed k-space data, splitting (209) the k-space data into one or more data chunks, wherein each data chunk of the data chunks comprises a set of one or more samples having a set of k-space coordinates, for each set of k-space coordinates of the one or more sets of coordinates, selecting (211), from the delta k-space data, a residual data set having the set of k-space coordinates, inputting (213) at least part of the data chunks and corresponding residual data sets to a trained machine learning model, thereby obtaining from the trained machine learning model probabilities of motion corruption for each of the data chunks of the acquired k-space.

FIG. 2

## Description

FIELD OF THE INVENTION

The invention relates to scanning imaging systems, in particular to a medical analysis system for enabling magnetic resonance images reconstruction.

BACKGROUND OF THE INVENTION

Magnetic resonance imaging (MRI) scanners rely on a large static magnetic field (B0) to align the nuclear spins of atoms as part of the procedure for producing images within the body of a patient. These images can reflect various quantities or properties of the subject. However, patient movement during the scans are often an imaging problem. Artefacts from patient movement are widely varied due to a dependence when during k-space filling the motion occurs.

SUMMARY OF THE INVENTION

Various embodiments provide for a medical analysis system, method, and computer program product, as described by the subject matter of the independent claims. Advantageous embodiments are described in the dependent claims.

Embodiments of the invention may provide for means for a magnetic resonance imaging method. A convolutional neural network (CNN) may detect motion corrupted data using a self-consistency matrix and measured k-space data. The self-consistency matrix may be measured using the inconsistencies between the measured k-space data from one data chunk/point and the reconstructed k-space data from all the acquired data. The measured self-consistency matrix and measured k-space data may be processed using the CNN to obtain a motion corrupted probability map. The CNN may process data per shot or data of all shots together. The method may further assign down weights to the CNN identified highly motion corrupted data chunk and higher weights to non-corrupted data chunk for image reconstruction.

In one aspect, the invention relates to a medical analysis system for reconstructing magnetic resonance images. The medical analysis system comprises a processor and at least one memory storing machine executable instructions. The processor is configured for controlling the medical analysis system. The medical analysis system comprises a trained machine learning model, wherein the trained machine learning model is configured for detecting motion corrupted data. Execution of the machine executable instructions causes the processor to: receive acquired k-space data of an object, reconstruct an image from the acquired k-space data, generate reconstructed k-space data from the reconstructed image, and determine delta k-space data as a difference between the acquired k-space data and the reconstructed k-space data. Execution of the machine executable instructions further causes the processor to split (or divide) the acquired k-space data into one or more data chunks, wherein each data chunk of the data chunks comprises a set of one or more samples having a set of respective k-space coordinates, and for each set of k-space coordinates of the one or more sets of k-space coordinates, select from the delta k-space data a residual data set comprising samples having the set of k-space coordinates. Execution of the machine executable instructions further causes the processor to input the whole or part of data chunks and corresponding residual data sets to the trained machine learning model, thereby obtaining an output from the trained machine learning model, wherein the output comprises probabilities of motion corruption for each of the input data chunks of the acquired k-space.

The present subject matter may employ self-consistency with a machine learning model to detect motion corrupted data. Using a combination of the self-consistency and machine learning model may be advantageous for the following reasons. The self-consistency concept alone may be successful in case of sufficient redundancy in the acquired data as motion corrupted data-chunks can easily be classified. The concept may, however, fail to detect the motion in case of high acceleration factors and/or large motion corruption. On the other hand, using only machine learning models such as neural networks, for detecting motion, by training the model with motion corrupted/non-corrupted images may have its own limitations. In particular, the accuracy of this technique may be pending the conclusiveness of the simulated data used for training. In addition, the models are trained for a specific anatomy which may not be generalizable to other anatomies and in order to have a well-trained model, multiple motion states need to be included in the training, which requires a large training data set with almost a conclusive set of all different types of motion. The present subject matter may solve these issues by applying a combination of the self-consistency and machine learning model on the k-space data instead of images. The self-consistency behaviour may be learnt for k-space data of low redundancy using a machine learning model, such that also non-obvious inconsistent data chunks can be detected. The motion detection may therefore be more robust to less redundant data compared to a conventional method.

The present subject matter may enable an accurate detection of motion corrupted data in acquired raw data. This may enable an efficient application of motion prevention and correction techniques. In particular, the present subject matter may enable to reduce the effects of motion which are particularly insidious, as motion reduces spectral quality and diagnostic value, but often does not cause distinctive artefacts and so can go unnoticed. By contrast to an ad-hoc method for detecting motion corrupted data, using machine learning models may further increase the detection accuracy of motion corrupted data.

The object may be a target volume in a subject. The subject may, for example, be imaged or scanned by an MRI system to acquire the k-space data. The acquired k-space data may be used to generate an image of the target volume in the subject. The target volume may, for example, be the head or heart.

The term "k-space," as used herein, may refer to an array of numbers (a matrix) representing spatial frequencies in an MR image. The k-space may be the 2D or 3D Fourier transform of an MR image.

The term "machine learning" (ML) refers to a computer algorithm used to extract useful information from training data sets by building probabilistic models (referred to as machine learning models or "predictive models") in an automated way. Machine learning algorithms build a mathematical model based on sample data, known as "training data", in order to make predictions or decisions. The machine learning may be performed using a learning algorithm such as supervised or unsupervised learning, [clustering, classification, linear regression,] reinforcement algorithm, self learning, etc. The machine learning may be based on various techniques such as clustering, classification, linear regression, support vector machines, neural networks, etc. A "model" or "predictive model" may for example be a data structure or program such as a neural network, a support vector machine, a decision tree, a Bayesian network etc. The model is adapted to predict an unmeasured value (e.g. which tag corresponds to a given token) from other, known values and/or to predict or select an action to maximize a future reward. According to one example, the machine learning model is a deep learning model.

The term "k-space coordinate" refers to a k-space coordinate of a sample (or data point or sampling point) of the acquired k-space data. The k-space coordinate is a k-space location. A k-space coordinate k (t) may, for example, be defined as

$$k(t) = \gamma \int_0^t G(t)\, dt,$$

where $\gamma$ is the gyromagnetic ratio and $G(t)$ is the gradient vector, describing the strength of the magnetic gradients along the $x, y,$ and/or $z$ axes at time $t$, and 0 refers to a starting time. The k-space coordinate $k(t)$ may, for example, be a 2-Dimenaionsl k-space coordinate e.g. $\{kx(t), ky(t)\}$ or a 3-Dimenaionsl k-space coordinate e.g. $\{kx(t), ky(t), kz(t)\}$ if a 2D or a 3D gradient magnetic field G(t) is applied respectively.

A data chunk of the acquired k-space data may comprise one or more samples, wherein each sample of the one or more samples has a respective k-space coordinate in k-space. Thus, each data chunk of the acquired k-space data may be associated with a respective set of one or more k-space coordinates of the samples of the data chunk. The samples of each data chunk of the acquired k-space data may be connected samples. The connected samples may be contiguous in acquisition time (and thus connected in time), or non-contiguous but connected with certain physiological phenomenon.

According to one embodiment, execution of the machine executable instructions further triggers the processor to process the input for one residual data set and one data chunk having the same set of k-space coordinates to the trained machine learning model. The actual measured and reconstructed k-space is provided as input because the residuals are a function of the actual object. The same trained machine learning model may be fixed/used for different systems, different objects and/or different types of movements. This may enable an efficient and universal usage of the trained machine learning model without having to retrain the model for each use case.

Inputting a residual data set and corresponding data chunk to the trained machine learning model comprises processing the input for the residual data set and corresponding data chunk to the trained machine learning model.

According to one embodiment, execution of the machine executable instructions further causes the processor to perform the inputting by repeatedly inputting one residual data set and one data chunk having the same set of k-space coordinates to the trained machine learning model until all selected residual data sets are processed.

According to one embodiment, execution of the machine executable instructions further causes the processor to perform the inputting by inputting multiple residual data sets and associated/corresponding multiple data chunks to the trained machine learning model. A data chunk corresponds with a residual data set as they have the same k-space coordinates. This may enable to obtain all motion corrupted data in a single output of the trained machine learning model. This may enable a flexible and easy usage of the trained model.

According to one embodiment, the set of k-space contiguous samples of a data chunk are contiguous with respect to their acquisition time or related by certain physiological measurements. This may enable a flexible and efficient sampling of k-space and may enable to quantify different types of motions such as rotations and translations of the object.

According to one embodiment, the acquired k-space data results from subjecting the object to a number K>=1 of shots of a predefined pulse sequence, wherein each data chunk of the data chunks comprises some or all samples of a respective single shot. This may enable to use the whole acquired k-space as a single chunk. This may enable a scalable solution as the input/output is given per shot only, hence it has a fixed number of inputs and one output.

According to one embodiment, the acquired k-space data results from subjecting the object to a number K of shots of a predefined pulse sequence, wherein each data chunk of the data chunks comprises samples of multiple shots selected according to a predefined sequence criterion. This may be advantageous as the object may move between positions during the scan, wherein that movement can be detected as the multiple shots can cover data of the positions. For example, in case of a periodic motion (e.g. a respiratory or cardiac motion), this embodiment may enable to capture a specific behaviour in this cyclic motion, as the data-points of this behaviour belong to multiple shots.

According to one embodiment, the trained machine learning model is a deep neural network, preferably a convolutional neural network (CNN). This may allow for varying input dimensions and may thus enable a flexible motion detection method.

According to one embodiment, execution of the machine executable instructions further causes the processor to use the output of the trained machine learning to generate a weighting map. The weighting map comprises a weight for each set of k-space coordinates of the one or more sets of k-space coordinates, wherein the weight indicates whether k-space data having the set of k-space coordinates is corrupted with motion. For example, down weights may be assigned to highly motion corrupted data chunks and higher weights to non-corrupted data chunks.

According to one embodiment, execution of the machine executable instructions further causes the processor to use the weighting map in an iterative re-weighted least squares coil combination scheme for reconstructing a motion corrected image. For example, when a data-chunk is highlighted as potentially inconsistent (i.e. potentially motion-corrupted), it can be mitigated or removed during the reconstruction, for instance during the coil combination process. This weighting map can be employed in the iterative re-weighted least squares coil combination scheme similar to, for instance, iterative compressed sense reconstruction or iterative sense reconstruction.

According to one embodiment, execution of the machine executable instructions further causes the processor to select sets of k-space samples that have weights higher than a predefined threshold. The threshold may be also iteratively defined during the optimization process. The k-space data representing the selected sets may be used for reconstructing a motion corrected image. For example, the rejection of the sets with lower weights may aim to cancel either an abrupt type of motion or just certain parts of a cyclic motion.

According to one embodiment, execution of the machine executable instructions further causes the processor to: receive a training data set. The training data set comprises data chunks, wherein each data chunk is labelled as being motion corrupted or not. The machine learning model may be trained using the training data set and the trained machine learning model may be provided. The machine learning model may be trained in a supervised manner, using chunks of data which are labelled as being motion corrupted or not. This embodiment may enable to use chunk-based self-consistency measure as a learning criterion for the model in contrast to relying on the images. The trained model may be independent of the anatomy and of the type of motion because the relation between the object signal in k-space and the residuals is expected to be generic information which is valid for all protocols/objects.

In another aspect, the invention relates to a magnetic resonance imaging, MRI, system comprising the system of any of the preceding embodiments. The MRI system is configured to acquire the k-space data.

In one aspect, the invention relates to a method comprising receiving acquired k-space data of an object, reconstructing an image from the acquired k-space data, generating reconstructed k-space data from the reconstructed image, determining delta k-space data as a difference between the acquired k-space data and the reconstructed k-space data, splitting the acquired k-space data into one or more data chunks, wherein each data chunk of the data chunks comprises a set of one or more samples having a set of k-space coordinates, for each set of k-space coordinates of the one or more sets of coordinates, selecting, from the delta k-space data, a residual data set having the set of k-space coordinates, inputting at least part of the data chunks and corresponding residual data sets to a trained machine learning model, thereby obtaining from the trained machine learning model probabilities of motion corruption for each of the input data chunks. The at least part of the input data chunks and corresponding residual data sets is the whole or part of data chunks of the acquired k-space data and corresponding residual data sets.

In another aspect, the invention relates to a computer program product comprising machine executable instructions for execution by a processor, wherein execution of the machine executable instructions causes the processor to the methods of any of the preceding embodiments.

It is understood that one or more of the aforementioned embodiments of the invention may be combined as long as the combined embodiments are not mutually exclusive.

BRIEF DESCRIPTION OF THE DRAWINGS

In the following preferred embodiments of the invention will be described, by way of example only, and with reference to the drawings in which:

Fig. 1 is a schematic diagram of a medical analysis system in accordance with the present subject matter,
Fig. 2 is a flowchart of a method for detecting MRI motion corrupted data in accordance with an example of the present subject matter,
Fig. 3 is a flowchart of a method for training a machine learning model in accordance with an example of the present subject matter,
Fig. 4A depicts a diagram illustrating a pre-processing method in accordance with the present subject matter,

Fig. 4B depicts a diagram illustrating an inference of a CNN in accordance with the present subject matter,

Fig. 5 shows a cross-sectional and functional view of an MRI system.

## DETAILED DESCRIPTION OF THE EMBODIMENTS

In the following, like numbered elements in the figures are either similar elements or perform an equivalent function. Elements which have been discussed previously will not necessarily be discussed in later figures if the function is equivalent.

Various structures, systems and devices are schematically depicted in the figures for purposes of explanation only and so as to not obscure the present invention with details that are well known to those skilled in the art. Nevertheless, the attached figures are included to describe and explain illustrative examples of the disclosed subject matter.

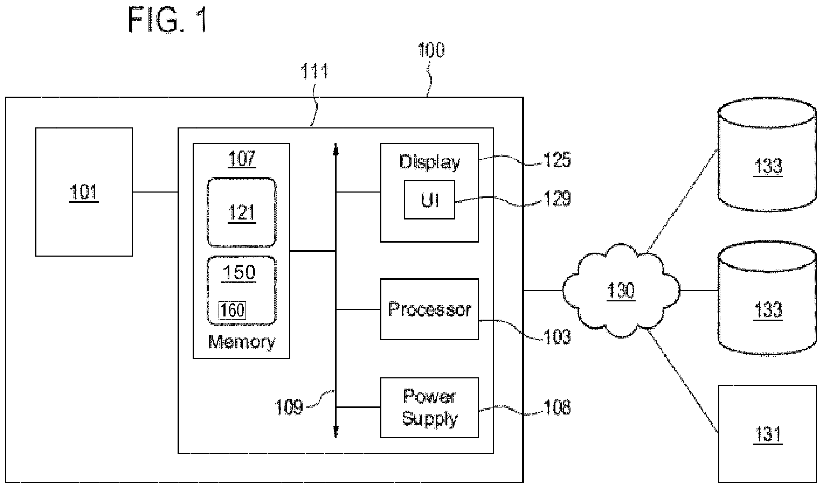

Fig. 1 is a schematic diagram of a medical analysis system 100. The medical analysis system 100 comprises a control system 111 that is configured to connect to a scanning imaging system (or acquisition component) 101. The control system 111 comprises a processor 103, a memory 107 each capable of communicating with one or more components of the medical analysis system 100. For example, components of the control system 111 are coupled to a bidirectional system bus 109.

It will be appreciated that the methods described herein are at least partly non-interactive, and automated by way of computerized systems. For example, these methods can further be implemented in software 121, (including firmware), hardware, or a combination thereof. In examples, the methods described herein are implemented in software, as an executable program, and is executed by a special or general-purpose digital computer, such as a personal computer, workstation, minicomputer, or mainframe computer.

The processor 103 is a hardware device for executing software, particularly that stored in memory 107. The processor 103 can be any custom made or commercially available processor, a central processing unit (CPU), an auxiliary processor among several processors associated with the control system 111, a semiconductor based microprocessor (in the form of a microchip or chip set), a micro-processor, or generally any device for executing software instructions. The processor 103 may control the operation of the scanning imaging system 101.

The memory 107 can include any one or combination of volatile memory elements (e.g., random access memory (RAM, such as DRAM, SRAM, SDRAM, etc.)) and non-volatile memory elements (e.g., ROM, erasable programmable read only memory (EPROM), electronically erasable programmable read only memory (EEPROM), programmable read only memory (PROM).

Note that the memory 107 can have a distributed architecture, where various components are situated remote from one another, but can be accessed by the processor 103. Memory 107 may store an instruction or data related to at least one other constituent element of the medical analysis system 100.

The control system 111 may further comprise a display device 125 which displays characters and images and the like e.g. on a user interface 129. The display device 125 may be a touch screen display device.

The medical analysis system 100 may further comprise a power supply 108 for powering the medical analysis system 100. The power supply 108 may for example be a battery or an external source of power, such as electricity supplied by a standard AC outlet.

The scanning imaging system 101 may comprise at least one of MRI, CT and PET-CT imagers. The control system 111 and the scanning imaging system 101 may or may not be an integral part. In other terms, the control system 111 may or may not be external to the scanning imaging system 101.

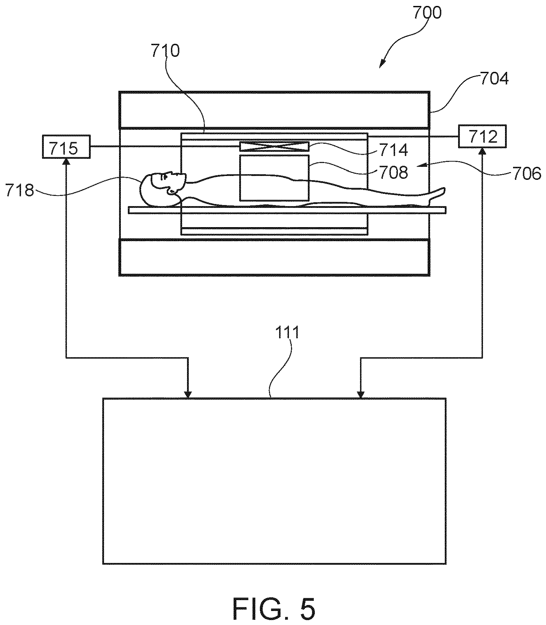

The scanning imaging system 101 comprises components that may be controlled by the processor 103 in order to configure the scanning imaging system 101 to provide image data to the control system 111. The configuration of the scanning imaging system 101 may enable the operation of the scanning imaging system 101. The operation of the scanning imaging system 101 may, for example, be automatic. Fig. 5 shows an example of components of the scanning imaging system 101 being an MRI system.

The connection between the control system 111 and the scanning imaging system 101 may for example comprise a BUS Ethernet connection, WAN connection, or Internet connection etc.

In one example, the scanning imaging system 101 may be configured to provide output data such as images in response to a specified measurement. The control system 111 may be configured to receive data such as MR image/k-space data from the scanning imaging system 101. For example, the processor 103 may be adapted to receive information (automatically or upon request) from the scanning imaging system 101 in a compatible digital form so that such information may be displayed on the display device 125. Such information may include operating parameters, alert notifications, and other information related to the use, operation and function of the scanning imaging system 101.

The medical analysis system 100 may be configured to communicate via a network 130 with other scanning imaging systems 131 and/or databases 133. The network 130 comprises for example a wireless local area network (WLAN) connection, WAN (Wide Area Network) connection LAN (Local Area Network) connection or a combination thereof. The databases 133 may comprise information relates to patients, scanning imaging systems, anatomies, scan geometries, scan parameters, scans etc. The databases 133 may for example comprise an electronic medical record (EMR) database comprising patients' EMR, Radiology Information System database, medical image database, PACS, Hospital Information System database and/or other databases comparing data that can be used for planning a scan geometry. The databases 133 may, for example, comprise training datasets for the training performed by the present subject matter.

The memory 107 may further comprise an artificial intelligence (AI) component 150. The AI component 150 may or may not be part of software component 121. The AI component 150 may, for example, comprise a trained machine learning model 160. The trained machine learning model 160 may be configured to receive at least a data chunk and to output a probability for each input data chunk to be motion corrupted.

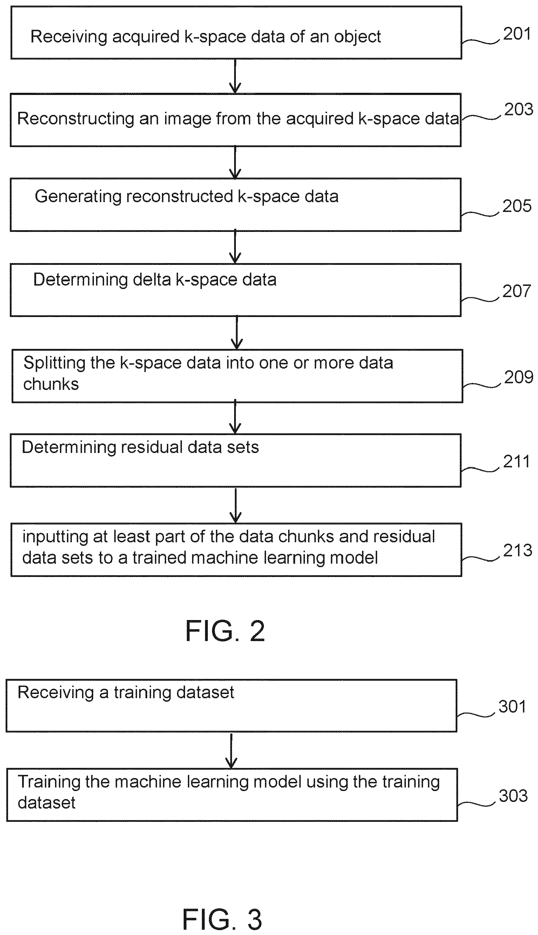

Fig. 2 is a flowchart of a method for detecting motion corrupted data. The method may, for example, be performed by a computer system such as control system 111 described with reference to Fig. 1.

Acquired k-space data m of an object may be received in step 201. In one example, the k-space data may be received from a remote MRI system. The MRI system may, for example, be remotely connected to the computer system via a network such as Internet. This may be advantageous as it may enable a centralized processing of acquired k-space data. For example, the method of Fig. 2 may be repeated for each received k-space data acquired by different remote MRI systems. In another example, the k-space data may be acquired by an MRI system which forms an integral part with the computer system. This may enable an autonomous and distributed processing of the acquired data.

In one example, the k-space data may be acquired in accordance with a single shot acquisition so that the entire k-space matrix is filled in one execution of the pulse sequence. The shot is a unit representing the data collection that is performed by the excitation pulse of one time. In another example, the k-space data may be acquired in accordance with a multi-shot acquisition so that more than one execution of the pulse sequence is performed to collect all the desired k-space data of the k-space matrix. A data chunk may be part of data of a single shot or may comprise selected data points that belong to different shots based on certain sequence criterion. For example, in case of a periodic motion of the object (e.g. respiratory or cardiac motion), using different shots may enable to capture specific behavior in the cyclic motion. In one example, each data chunk of the one or more data chunks may be assigned an index indicative of the set of k-space coordinates that is represented by the data chunk.

Upon receiving the acquired k-space data, a pre-processing method may be applied on the acquired k-space data. The pre-processing method comprises steps 203 to 211.

A MR image p may be reconstructed in step 203 from the acquired k-space data. For that, an inverse Fourier transformation may, for example, be performed on the k-space data. The k-space data may be transformed into frequency-domain data which may, for example, be a two-dimensional (2-D) or three-dimensional (3-D) data set. Exemplary image reconstruction methods may include parallel imaging, Fourier reconstruction, constrained image reconstruction, compressed sensing, or the like, or a variation thereof, or any combination thereof.

Reconstructed k-space data $kr$ may be generated in step 205 from the reconstructed image p. The reconstructed k-space data may be generated by multiplying an encoding matrix $E$ with the reconstructed image $p$: $kr=E.p$. The reconstructed k-space data may be provided as a reconstructed k-space matrix having the same size as the acquired k-space matrix.

Delta k-space data $R$ may be determined in step 207. The delta k-space data $R$ may be obtained as the difference between the acquired k-space data $m$ and the reconstructed k-space data $kr$: $R = m - E.p$. The delta k-space data may be provided as a self-consistency matrix. The self-consistency may be determined using motion residuals, where the motion residuals denote a difference between the reconstructed k-space $kr$ using all the acquired data and the measured or acquired k-space $m$.

The k-space data $m$ may, for example, be provided or stored as a k-space matrix or map, wherein each point or sample of the k-space data has its own k-space coordinates in the k-space matrix. The k-space data $m$ may be split (or divided) in step 209 into a number N of distinct data chunks $m^c$, where c=1, ... N and N>=1. Each data chunk of the data chunks may comprise samples that have a respective set of k-space coordinates $S^c$ in the k-space matrix. The set of k-space coordinates $S^c$ may be contiguous or non-contiguous coordinates in the k-space matrix. This may be advantageous as it may enable to detect movements at a higher degree of freedom. Each data chunk of the data chunks may comprise one or more data points.

Assuming for simplification of the description that N=2, the data chunk $m^1$ may comprise one data point having a k-space coordinate $S^1$ e.g. (kx1, ky1), and the data chunk $m^2$ may comprise multiple data points having a set of k-space coordinate $S^2$ e.g. (kx2, ky2) and (kx4, ky4).

For each set of k-space coordinates $S^c$ of the N sets of k-space coordinates, the corresponding residual data set $R^c$ is selected in step 211 from the delta k-space data $R$. The selected residual data set $R^c$ comprises samples having the set of k-space coordinates $S^c$. The residual data set may be referred to as a data chunk residual $R^c$. Following the above example, a data point of the delta k-space data R having the coordinates (kx1, ky1) of the set $S^1$ may be selected and data points of the delta k-space data $R$ having the coordinates (kx2, ky2) and (kx4, ky4) of the set $S^2$ may be selected. In one example, the data chunk residual $R^c$ of data chunk c may be defined using a selection mask $M^c$ to select all the data points from chunk c as follows: $R^c = M^c. (m - E.p)$.

The selection matrix of data chunk residual $R^c$ may be determined using the set of k-space coordinates of samples of data chunk c. The selection matrix may, for example, be user defined. The data chunk residual $R^c$ may vary in size, for instance: it is a 3D matrix for 2D multicoil acquisition and a 4D matrix for a 3D multicoil acquisition.

The execution of the pre-processing method may thus result in N residual data sets $R^c$ and N corresponding measured data sets $m^c$. That is, N pairs $(R^1, m^1) ... (R^N, m^N)$ of residual data sets and measured data sets. Following the above example, the execution of the pre-processing method may result in two pairs $(R^1, m^1)$ and $(R^2, m^2)$ associated with the sets of k-space coordinates $S^1$ and $S^2$ respectively.

In step 213, at least part of the N pairs may be input to the trained machine learning model 160, in order to obtain data indicative of motion corrupted data points. Step 213 may enable an inference of the trained machine leaning model. Step 213 may be performed in accordance with one or more inference examples.

In a first inference example, one pair of the N pairs e.g. $(R^2, m^2)$ is input to the trained machine learning model. The trained machine learning model may provide an output indicating a probability that the data chunk $m^2$ is motion corrupted.

In a second inference example, all N pairs may be provided as input to the trained machine learning model. The trained machine learning model may output N probabilities $p^c$, each indicating the probability that a respective data chunk $m^c$ is motion corrupted.

The inference example used in step 213 may be chosen based on the training method (e.g. as described with reference to Fig. 3) used for training the machine learning model 160.

The method of Fig. 2 may, for example, automatically be executed upon receiving the acquired k-space data.

Fig. 3 is a flowchart of a method for training a machine learning model 160 in accordance with an example of the present disclosure. For exemplification purpose the machine learning model may be a CNN

In step 301, a training set may be received e.g. by the control system 111. For example, the training set may be obtained from one or more sources. For example, the training set may be retrieved by the control system 111 from the databases 133.

In order to enable the first inference example, the training set may comprise entries, wherein each entry of the entries comprises a pair $(R^c, m^c)$ of a data chunk and a residual data set having same k-space coordinates and a label $L^c$ indicating whether the data chunk $m^c$ is motion corrupted. Thus, each entry of the of the training set may comprise a triplet $(R^c, m^c, L^c)$. In this case, the input layer of the CNN may comprise a number of nodes equal to the number of coordinates of the set of coordinates times 2.

In order to enable the second inference example, the training set may comprise entries, wherein each entry comprises a triplet $(R^1... R^N, m^c...m^N, L^1... L^N)$ of all N data chunks, associated residual sets and the labels of each of the data chunks. In this case, the number of all coordinates of the samples of the N of data chunks defines the number of nodes in the input layer of the CNN. For example, each data point of the pair of data chunk and the residual data set may be associated with a respective node of the CNN

In step 303, the machine learning model 160 may be trained using the received training set. The CNN may, for example, comprise groups of weights e.g. weights from the input layer to a first hidden layer, from first to second hidden layer etc. Before the training of the CNN, the weights may be initialized by random numbers or values. The training may be performed in order to search optimization parameters (e.g. weights and bias) of the CNN and minimize the classification error or residuals. For example, the training set is used as input for feeding forward the CNN. This may enable to compute data loss in the output layer of the CNN e.g. by a loss function (cost function). The data loss measures the compatibility between a predicted task and the ground truth label. After getting data loss, the data loss may be minimized by changing the weights and bias of the CNN. This may for example be performed by back-propagating the loss into every layers and neuron by gradient descent.

In one example, the training set of step 301 may continuously be enhanced using additional data. For example, the training set may be updated by adding the processed data chunks associated probabilities. Additionally or alternatively, the training set may be updated by adding further triplets. Step 303 may regularly be repeated e.g. as soon as the training set has been updated. And the retrained CNN may, for example, be used instead of the trained CNN in the method of Fig. 2.

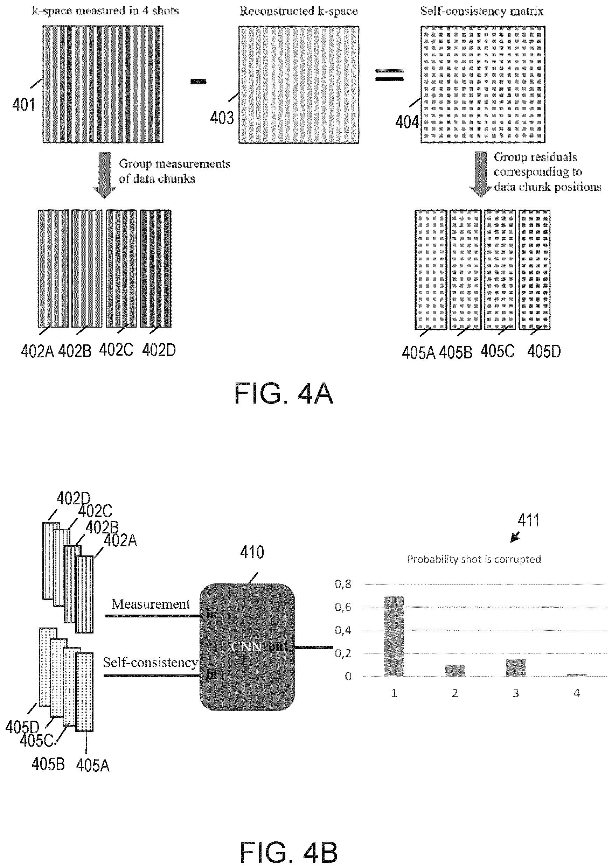

Fig. 4A depicts a block diagram illustrating steps of the pre-processing method. For example, the object is subjected to a number K=4 of shots of a predefined pulse sequence. The number of shots K may be equal to the number of data chunks N e.g. each data chunk may comprise samples of a respective shot. In another example, number of shots K may be different from the number of data chunks N e.g. each data chunk may comprise samples of a more than one shot. In this example, the acquired k-space data 401 is split into four distinct data chunks 402A, 402B, 402C and 402D, wherein each data chunk comprises samples of a respective shot of the four shots.

Reconstructed k-space data 403 is determined by reconstructing an image from the acquired k-space data, and converting the image to k-space. The reconstructed k-space data 403 is subtracted from the acquired k-space data 401 to get a self-consistency matrix 404. The residuals or data points of the self-consistency matrix 404 are grouped into four residual data sets 405A, 405B, 405C and 405D corresponding to the data chunk positions of data chunks 402A, 402B, 402C and 402D respectively. This may result in four pairs (402A, 405A), (402B, 405B), (402C, 405C), (402D, 405D) of data chunks and associated residual data sets. The data chunk and the residual data set of each pair of the pairs have the same set of k-space coordinates.

The acquired k-space and the self-consistency matrix are subsequently fed to a CNN 410 as shown in Fig. 4B, which processes the chunks of data one-by-one. As shown in Fig. 4B, each pair of the four pairs is provided as input at once. For each processed chunk a probability that it is motion corrupted is given in a probability map 411. For the network configuration there are multiple options: one for which the CNN processes data per shot and one for which the CNN processes the data of all shots together. The latter may contain more information for the network to use to detect motion but may also less scalable because the input and output size is varying from one scan to another (e.g. different number of shots results in a different number of inputs/outputs). In this case, the CNN may be trained per use case. In case the CNN processes the data per shot which the network has a fixed number of inputs/outputs. For that case, a wide variety of scans can be handled with one single network.

Fig. 5 illustrates a magnetic resonance imaging system 700 as an example of the medical analysis system 100. The magnetic resonance imaging system 700 comprises a magnet 704. The magnet 704 is a superconducting cylindrical type magnet with a bore 706 in it. The use of different types of magnets is also possible; for instance, it is also possible to use both a split cylindrical magnet and a so called open magnet or a sealed magnet. A split cylindrical magnet is similar to a standard cylindrical magnet, except that the cryostat has been split into two sections to allow access to the iso-plane of the magnet. Such magnets may for instance be used in conjunction with charged particle beam therapy. An open magnet has two magnet sections, one above the other with a space in-between that is large enough to receive a subject 718 to be imaged, the arrangement of the two sections area similar to that of a Helmholtz coil. Inside the cryostat of the cylindrical magnet there is a collection of superconducting coils. Within the bore 706 of the cylindrical magnet 704 there is an imaging zone or volume or anatomy 708 where the magnetic field is strong and uniform enough to perform magnetic resonance imaging.

Within the bore 706 of the magnet there is also a set of magnetic field gradient coils 710 which is used during acquisition of magnetic resonance data to spatially encode magnetic spins of a target volume within the imaging volume or examination volume 708 of the magnet 704. The magnetic field gradient coils 710 are connected to a magnetic field gradient coil power supply 712. The magnetic field gradient coils 710 are intended to be representative. Typically, magnetic field gradient coils 710 contain three separate sets of coils for the encoding in three orthogonal spatial directions. A magnetic field gradient power supply supplies current to the magnetic field gradient coils. The current supplied to the magnetic field gradient coils 710 is controlled as a function of time and may be ramped or pulsed.

MRI system 700 further comprises an RF coil 714 at the subject 718 and adjacent to the examination volume 708 for generating RF excitation pulses. The RF coil 714 may include for example a set of surface coils or other specialized RF coils. The RF coil 714 may be used alternately for transmission of RF pulses as well as for reception of magnetic resonance signals e.g., the RF coil 714 may be implemented as a transmit array coil comprising a plurality of RF transmit coils. The RF coil 714 is connected to one or more RF amplifiers 715.

The magnetic field gradient coil power supply 712 and the RF amplifier 715 are connected to a hardware interface of control system 111. The memory 107 of control system 111 may for example comprise a control module. The control module contains computer-executable code which enables the processor 103 to control the operation and function of the magnetic resonance imaging system 700. It also enables the basic operations of the magnetic resonance imaging system 700 such as the acquisition of magnetic resonance data.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a 'circuit', 'module' or 'system'. Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, a data may be retrieved over a modem, over the internet, or over a local area network. Computer executable code embodied on a computer readable medium may be transmitted using any appropriate medium, including but not limited to wireless, wireline, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

A 'computer memory' or 'memory' is an example of a computer-readable storage medium. A computer memory is any memory which is directly accessible to a processor. A 'computer storage' or 'storage' is a further example of a computer-readable storage medium. A computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising 'a processor' should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the 'C' programming language or similar programming languages and compiled into machine executable instructions. In some instances, the computer executable code may be in the form of a high-level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It will be understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device'. A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, gear sticks, steering wheel, pedals, wired glove, dance pad, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bistable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word 'comprising' does not exclude other elements or steps, and the indefinite article 'a' or 'an' does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

LIST OF REFERENCE NUMERALS

| | |
|---|---|
| 100 | medical analysis system |
| 101 | scanning imaging system |
| 103 | processor |
| 107 | memory |
| 108 | power supply |
| 109 | bus |
| 111 | control system |
| 121 | software |
| 125 | display |
| 129 | user interface |
| 133 | databases |
| 150 | AI component |
| 160 | machine learning model |
| 201-213 | method steps |
| 301-303 | method steps |
| 401 | acquired k-space data |
| 402A-D | data chunks |
| 403 | reconstructed k-space data |
| 404 | self-consistency matrix |
| 405A-D | residual data sets |
| 410 | CNN |
| 411 | probability map |
| 700 | magnetic resonance imaging system |
| 704 | magnet |
| 706 | bore of magnet |
| 708 | imaging zone |
| 710 | magnetic field gradient coils |
| 712 | magnetic field gradient coil power supply |
| 714 | radio-frequency coil |
| 715 | RF amplifier |
| 718 | subject |

**Claims**

1. A medical analysis system (100) for enabling a magnetic resonance image reconstruction, the medical analysis system comprising a processor (103) and at least one memory (107) storing machine executable instructions, the processor being configured for controlling the medical analysis system (100), wherein execution of the machine executable instructions causes the processor (103) to:

   provide a trained machine learning model (160), the trained machine learning model (160) being configured for detecting motion corrupted data;

receive (201) acquired k-space data of an object;

reconstruct (203) an image from the acquired k-space data;

generate (205) reconstructed k-space data from the reconstructed image;

determine (207) delta k-space data as a difference between the acquired k-space data and the reconstructed k-space data;

split (209) the acquired k-space data into one or more data chunks, wherein each data chunk of the data chunks comprises a set of one or more samples having a set of k-space coordinates;

for each set of k-space coordinates of the one or more sets of k-space coordinates, select (211), from the delta k-space data, a residual data set having the set of k-space coordinates;

input (213) at least part of the data chunks and corresponding residual data sets to the trained machine learning model, thereby obtaining from the trained machine learning model probabilities of motion corruption for each of the input data chunks.

2. The system of claim 1, wherein execution of the machine executable instructions further causes the processor to perform the inputting by inputting one residual data set and one data chunk having the same set of k-space coordinates to the trained machine learning model.

3. The system of claim 1, wherein execution of the machine executable instructions further causes the processor to perform the inputting by repeatedly inputting one residual data set and one data chunk having the same set of k-space coordinates to the trained machine learning model until all selected residual data sets are processed.

4. The system of claim 1, wherein execution of the machine executable instructions further causes the processor to perform the inputting by inputting multiple residual data sets and associated multiple data chunks to the trained machine learning model.

5. The system of any of the preceding claims, wherein the set of k-space coordinates of a data chunk are contiguous with respect to their acquisition time or related by certain physiological measurements..

6. The system of any of the preceding claims, wherein the acquired k-space data results from subjecting the object to a number K>=1 of shots of a predefined pulse sequence, wherein each data chunk of the data chunks comprises some or all samples of a single shot.

7. The system of any of the preceding claims 1-5, wherein the acquired k-space data results from subjecting the object to a number K of shots of a predefined pulse sequence, wherein each data chunk of the data chunks comprises samples of multiple shots selected according to a predefined sequence criterion.

8. The system of any of the preceding claims, the trained machine learning model being a deep neural network, preferably a convolutional neural network, CNN

9. The system of any of the preceding claims, wherein execution of the machine executable instructions further causes the processor to use the output of the trained machine learning to generate a weighting map, the weighting map comprising a weight for each set of k-space coordinates of the one or more sets of k-space coordinates, the weight indicating whether k-space data having the set of k-space coordinates is corrupted with motion.

10. The system of claim 9, wherein execution of the machine executable instructions further causes the processor to use the weighting map in an iterative re-weighted least squares coil combination scheme for reconstructing a motion corrected image.

11. The system of claim 9, wherein execution of the machine executable instructions further causes the processor to select sets of k-space coordinates of the sets of k-space coordinates whose weights are higher than a predefined threshold, using the k-space data representing the selected sets for reconstructing a motion corrected image.

12. The system of any of the preceding claims, wherein execution of the machine executable instructions further causes the processor to: receive a training data set, the training data set comprising data chunks, each data chunk being labelled as being motion corrupted or not, training the machine learning model and providing the trained machine learning model.

13. A magnetic resonance imaging, MRI, system comprising the system of any of the preceding claims 1-12, the MRI system being configured to acquire the k-space data.

14. A method comprising:

receiving (201) acquired k-space data of an object;

reconstructing (203) an image from the acquired k-space data;

generating (205) reconstructed k-space data from the reconstructed image;

determining (207) delta k-space data as a difference between the acquired k-space data and the reconstructed k-space data;

splitting (209) the k-space data into one or more data chunks, wherein each data chunk of the data chunks comprises a set of one or more samples having a set of k-space coordinates;

for each set of k-space coordinates of the one or more sets of coordinates, selecting (211), from the delta k-space data, a residual data set having the set of k-space coordinates;

inputting (213) at least part of the data chunks and corresponding residual data sets to a trained machine learning model, thereby obtaining from the trained machine learning model probabilities of motion corruption for each of the input data chunks.

15. A computer program product comprising machine executable instructions for execution by a processor, wherein execution of the machine executable instructions causes the processor to perform at least part of the method of claim 14.

FIG. 1

| Receiving acquired k-space data of an object | 201 |
| Reconstructing an image from the acquired k-space data | 203 |
| Generating reconstructed k-space data | 205 |
| Determining delta k-space data | 207 |
| Splitting the k-space data into one or more data chunks | 209 |
| Determining residual data sets | 211 |
| inputting at least part of the data chunks and residual data sets to a trained machine learning model | 213 |

FIG. 2

| Receiving a training dataset | 301 |
| Training the machine learning model using the training dataset | 303 |

FIG. 3

k-space measured in 4 shots

401

Group measurements
of data chunks

402A 402B 402C 402D

Reconstructed k-space

403

Self-consistency matrix

404

Group residuals
corresponding to
data chunk positions

405A 405B 405C 405D

FIG. 4A

402D
402C
402B
402A

405D
405C
405B 405A

410

Measurement in

Self-consistency in

CNN out

411

Probability shot is corrupted

0,8
0,6
0,4
0,2
0

1       2       3       4

FIG. 4B

FIG. 5

Europäisches
Patentamt
European
Patent Office
Office européen
des brevets

# EUROPEAN SEARCH REPORT

Application Number

EP 20 17 4072

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BRADA, RAFI ET AL.: "Towards motion-robust MRI - Autonomous motion timing and correction during MR scanning using multi-coil data and a deep-learning neural network", INTERNATIONAL SOCIETY FOR MAGNETIC RESONANCE IN MEDICINE, ISMRM, 2030 ADDISON STREET, 7TH FLOOR, BERKELEY, CA 94704 USA, no. 4438, 26 April 2019 (2019-04-26), XP040711822, | 15 | INV. G01R33/565 G01R33/56 A61B5/055 G06N3/08 ADD. G01R33/561 |
| A | * sections "Synopsis", "Introduction" and "Methods"; figures 1,2 * | 1-14 | |
| X | KÜSTNER THOMAS ET AL: "Automated reference-free detection of motion artifacts in magnetic resonance images", MAGNETIC RESONANCE MATERIALS IN PHYSICS, BIOLOGY AND MEDICINE, SPRINGER, DE, GB, vol. 31, no. 2, 20 September 2017 (2017-09-20), pages 243-256, XP036467584, ISSN: 0968-5243, DOI: 10.1007/S10334-017-0650-Z [retrieved on 2017-09-20] | 15 | |
| A | * Abstract; page 246; figures 1-6 * | 1-14 | TECHNICAL FIELDS SEARCHED (IPC) G01R A61B G06N |
| X | ILKAY OKSUZ ET AL: "Deep Learning Based Detection and Correction of Cardiac MR Motion Artefacts During Reconstruction for High-Quality Segmentatio", ARXIV.ORG, CORNELL UNIVERSITY LIBRARY, 201 OLIN LIBRARY CORNELL UNIVERSITY ITHACA, NY 14853, 11 October 2019 (2019-10-11), XP081514302, | 15 | |
| A | * sections IV.B. and IV.C on p. 4; figures 3,4 * | 1-14 | |

The present search report has been drawn up for all claims

| Place of search | Date of completion of the search | Examiner |
|---|---|---|
| Munich | 5 October 2020 | Lebar, Andrija |

EPO FORM 1503 03.82 (P04C01)